# EUROPEAN PATENT SPECIFICATION

(11) **EP 4 380 657 B1**
(45) Date of publication and mention of the grant of the patent: **16.09.2026**
(21) Application number: 22851483.2
(22) Date of filing: 04.08.2022
(51) Int. Cl.: A61M 16/01, A61M 16/04, A61M 16/00, A61M 16/08, A61M 16/20

(54) **CONNECTOR PIECE FOR AN ANAESTHETIC BREATHING CIRCUIT**
ANSCHLUSSSTÜCK FÜR EINEN BEATMUNGSKREISLAUF MIT ANÄSTHESIE
PIÈCE RACCORD POUR UN CIRCUIT RESPIRATOIRE ANESTHÉSIQUE

(30) Priority: 05.08.2021 AU 2021104963; 10.11.2021 AU 2021903603
(43) Date of publication of application: 12.06.2024
(73) Proprietor: Dunlop, Colin, East Ryde, NSW 2113 (AU)
(72) Inventor: Dunlop, Colin, East Ryde, NSW 2113 (AU)
(74) Representative: Grünecker Patent- und Rechtsanwälte PartG mbB
(86) International application number: PCT/AU2022/050838
(87) International publication number: WO 2023/010172

(56) References cited:
- WO-A1-2011/122964
- WO-A1-2014/193847
- WO-A1-2018/236228
- WO-A2-2016/022934
- DE-A1- 3 623 367
- US-A1- 2008 011 305
- US-A1- 2015 000 652
- US-B2- 8 739 779
- LONGHITANO GUILHERME ARTHUR ET AL: "The role of 3D printing during COVID-19 pandemic: a review", vol. 6, no. 1, 24 November 2020 (2020-11-24), DE, pages 19 - 37, XP093033581, ISSN: 2363-9512, Retrieved from the Internet <URL:http://link.springer.com/article/10.1007/s40964-020-00159-x/fulltext.html> [retrieved on 20241014], DOI: 10.1007/s40964-020-00159-x
- LONGHITANO GUILHERME ARTHUR, NUNES GUILHERME BITENCOURT, CANDIDO GEOVANY, DA SILVA JORGE VICENTE LOPES: "The role of 3D printing during COVID-19 pandemic: a review", PROGRESS IN ADDITIVE MANUFACTURING, SPRINGER, DE, vol. 6, no. 1, 1 February 2021 (2021-02-01), DE , pages 19 - 37, XP093033581, ISSN: 2363-9512, DOI: 10.1007/s40964-020-00159-x

## Description

### TECHNICAL FIELD

This invention relates to a connector piece for connecting the breathing tubes of an anaesthetic breathing circuit to an endotracheal tube for use with a mammal. In particular, the present invention is described with reference to a connector piece for connecting the breathing tubes of an anaesthetic breathing circuit to an endotracheal tube for use with a small mammal such as a cat, small dog or infant during surgery or other procedures.

### BACKGROUND

Mechanical dead space is dead space in an apparatus in which the breathing gas must flow in both directions as the user breathes in and out, increasing the necessary respiratory effort to get the same amount of usable air or breathing gas, and risking accumulation of carbon dioxide from shallow breaths. It is in effect an external extension of the physiological (anatomical) dead space. In anaesthetic breathing circuits, it is preferable to minimise the mechanical dead space between a mammal and the breathing tubes of the anaesthetic breathing circuit. The effect of mechanical dead space is of particular concern when anaesthetizing smaller mammals, including humans, which weigh 15 kilograms or less.

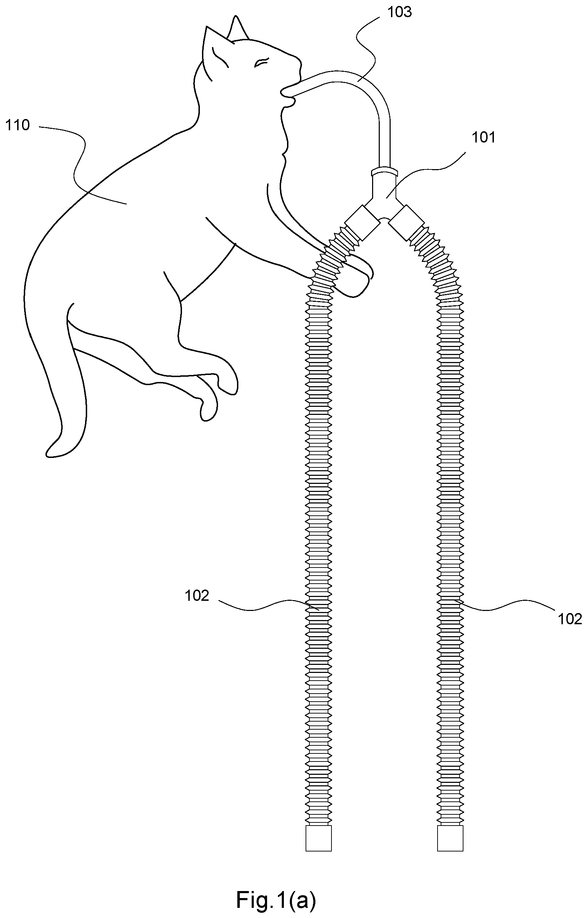

There are typical scenarios where small mammals, such as cats, small dogs and human infants undergo procedures requiring anaesthesia, where mechanical dead space is a problem. Standard paediatric Y-piece connectors are known for connecting the breathing tubes of an anaesthetic breathing circuit to an endotracheal tube for use in small mammals. For example, standard paediatric 15mm (outside diameter) Y-piece connectors 101 are shown in Figs 1(a) and (1b) respectively, for a cat 110 and an infant 111 are shown.

In Fig 1(a) cat 110 is shown on its side, as if positioned for a dental procedure (animals need to be anaesthetised for dentistry, a very common procedure) or for surgery of the head, the eyes, or the ears, with the breathing tubes 102 running towards the anaesthetic equipment (not shown) which would be located toward the rear or side of the surgery table (not shown). The anaesthetist (not shown) would sit next to the anaesthesia machine looking towards the feet and tail of cat 110. The dentist or surgeon (also not shown) would stand or be seated at the head end of the surgery table. All connections are shown to enable the breathing circuit tube direction to facilitate this positioning. To achieve the correct breathing circuit tube direction, the endotracheal tube 103, (typically having a 5mm inside diameter) and which may typically be made of vinyl, needs to extend further than normal (typically extends 3cm; in this case an additional 4cm = total 7cm protruding from the mouth of cat 110). This additional 4cm endotracheal tube dead space is undesirable.

The "dead space" is the volume within which the expired gas (from cat 110) that contains CO₂, must be rebreathed before "fresh gas" not containing CO₂ is breathed in. In addition to adding additional mechanical dead space, the extended and bent vinyl endotracheal tube 103 is liable to having its wall kink, thereby occluding the airway.

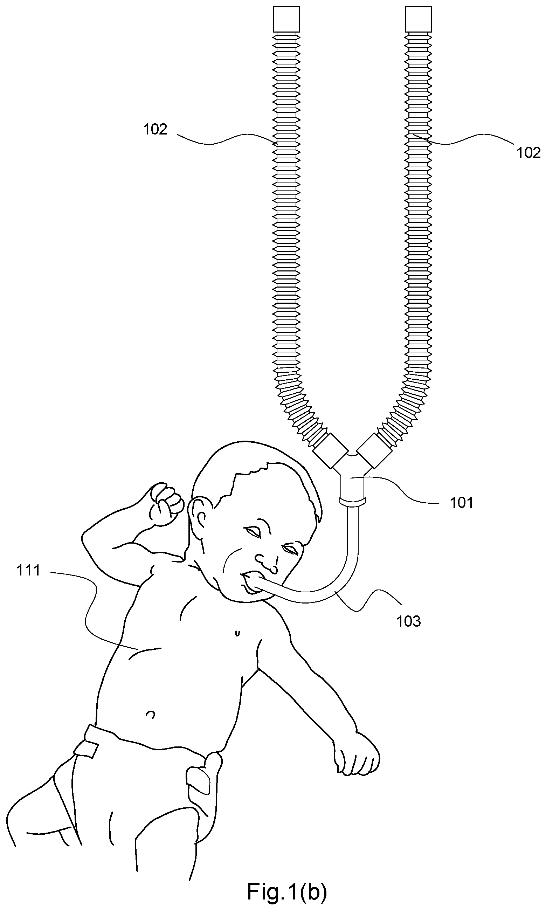

In Fig. 1 (b), infant 111 is shown on its back as if positioned for abdominal surgery with the breathing tubes 102 running towards the anaesthetic equipment (not shown) which would be at the "head" of the surgery table. The anaesthetist (not shown) would sit next to the anaesthesia machine looking toward the infant's head, the surgeon (also not shown) would stand to one side of the abdomen of infant 111. All connections are shown to enable the breathing circuit tube direction to facilitate this positioning. In a similar fashion to the arrangement of cat 110 in Fig. 1(a), to achieve the correct breathing circuit tube direction, the endotracheal tube 103, (typically having a 5mm inside diameter) and which may also typically be made of vinyl, needs to extend further than normal (typically extends 3cm; in this case an additional 4cm = total 7cm protruding from the mouth of infant 111). Like that of the example of cat 110 in Fig. 1(a), this additional 4cm endotracheal tube dead space is undesirable.

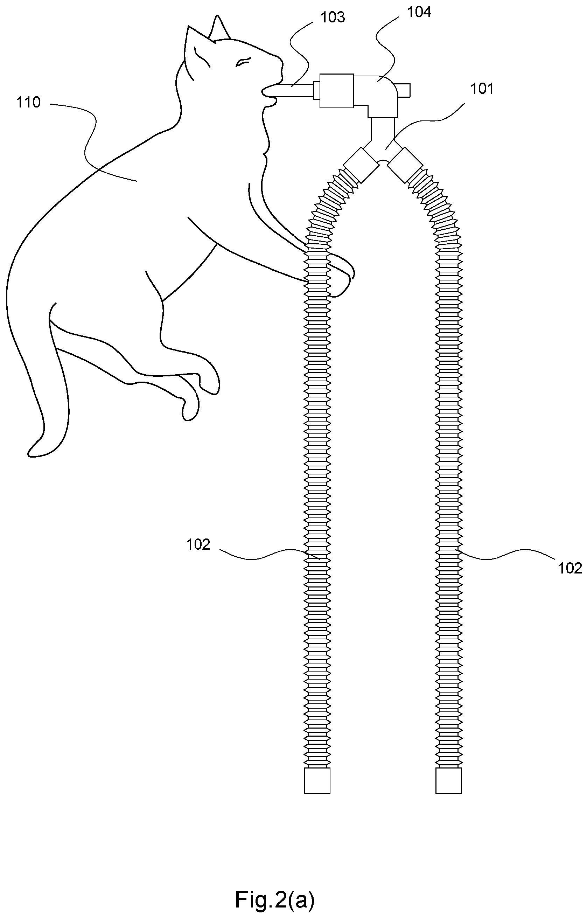

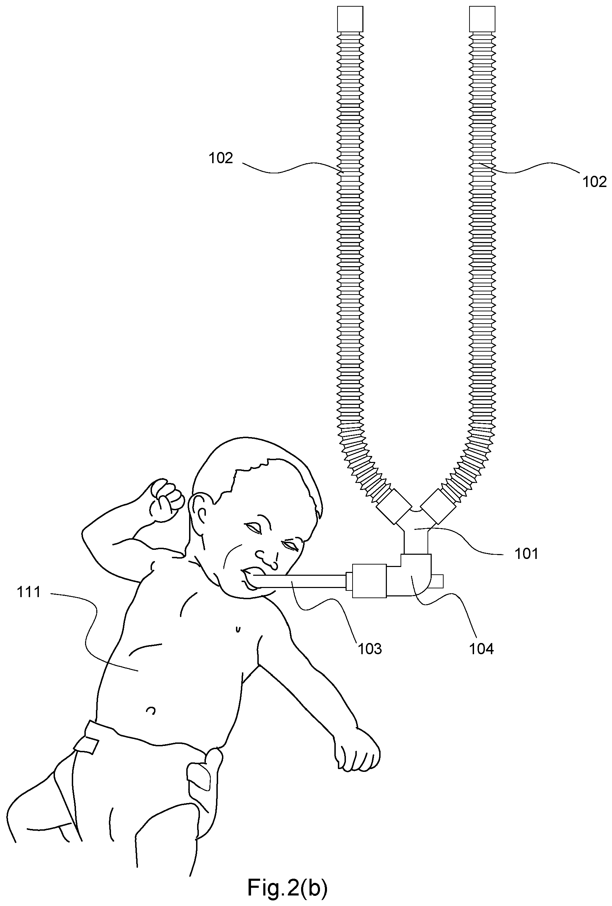

To avoid or minimize the risk of kinking the vinyl endotracheal tube 103, you could employ the addition of an elbow 104 between the Y-piece connector 101 and the endotracheal tube 103 as shown for the cat 110 and infant 111 in Figs 2(a) and 2(b), respectively. However, whilst the use of elbow 104 reduces the risk of kinking endotracheal tube 103, it is at the expense of adding substantially more mechanical dead space of the additional connector (elbow 104), which is undesirable. This additional mechanical dead space also dilutes the concentration of inspired anaesthetic gas, so the patient, say cat 110, is at risk for inadvertent "wake up" during the procedure.

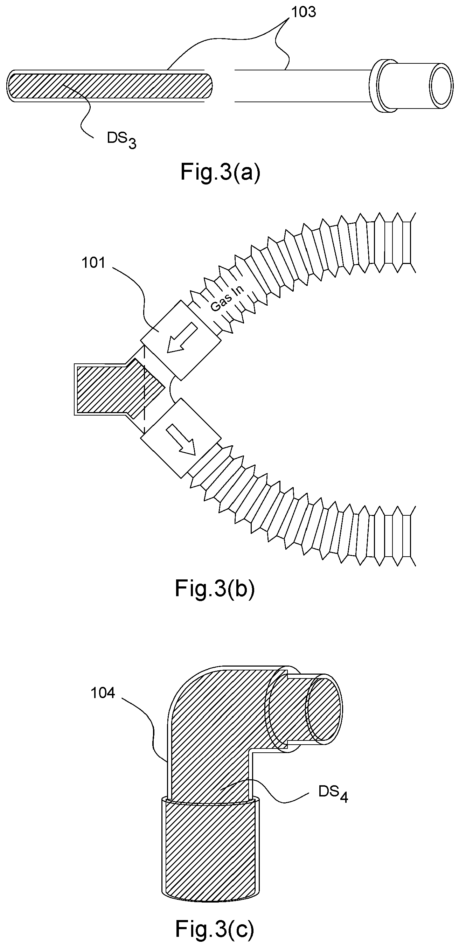

The certain dead space volumes of the prior art endotracheal tube 103 and ninety-degree (90°) elbow 104 are shown as the grey shaded volumes DS₃ and DS₄ in Figs 3(a) and 3(c) respectively. These areas are referred to in comparison calculation within the description of the present specification.

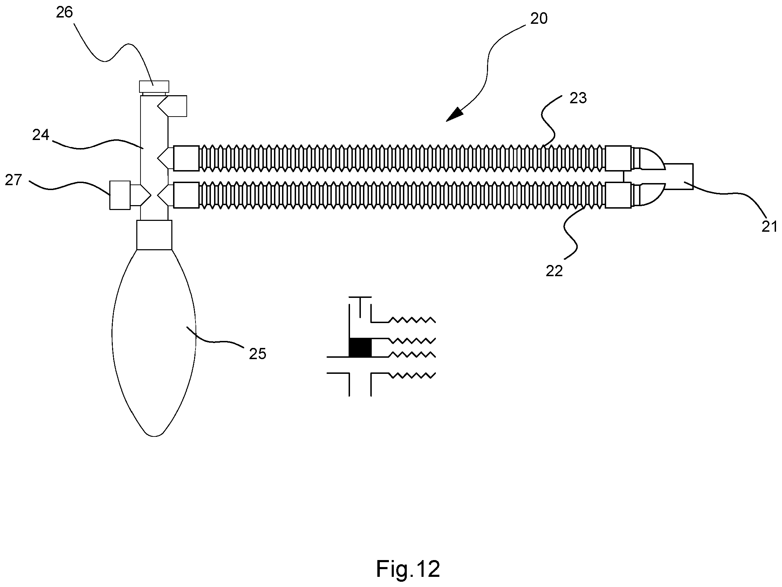

The above-described Y-piece connectors are used in veterinary anaesthesia in both "rebreathing" and "non-rebreathing" circuits. One prior art non-rebreathing circuit is the parallel Lack circuit described with reference to Fig. 12. In this parallel Lack circuit 20, an adult Y-piece 21 is shown at the patient end, intended to connect to an endotracheal tube (not shown) of a mammal. Extending from Y-piece 21 are two twenty mm ID parallel corrugated tubes, identified in Fig. 1 as lower fresh gas inspiratory tube 22 and upper reservoir tube 23 which extend to circuit block 24 at the "vaporiser end" of circuit 20. Circuit block 24 is fitted with a reservoir bag 25 and a waste gas outlet 26 and has an inlet 27 through which fresh gas (containing anaesthetic agent) is delivered from a vaporiser (not shown). Circuit block 24 is blanked off between the inlet 27 side and waste outlet 26 so that there is no connection therebetween within block 24. During inspiration, exhalation valve (port) 26 closes and the patient inspires fresh gas from the lower inspiration tube 22. During expiration the patient expires into reservoir tube 23. Towards the end of expiration, reservoir bag 25 fills and positive pressure opens valve 26, allowing expired gas to escape via reservoir tube 23. During the "expiratory pause", fresh gas washes the expired gas out of reservoir tube 23, filling it with fresh gas

Two commercially available parallel Lack circuits, are offered by "Burtons veterinary" of the United Kingdom. The first identified as the "Non-disposable Lack circuit" is indicated to suit veterinary patients greater than 10kg, whilst the second identified as the "Mini-Lack Anaesthetic Breathing System" is indicated for bodyweights in the range of 1kg-10kg. Whilst the promotional material of the "Mini-Lack Anaesthetic Breathing System" indicates that it reduces the gas flowrate to be suitable for smaller mammals, this device still uses a conventional open-close pop-off waste gas valve outlet, and because the flow rate is still too high, it does not address the issue of hypothermia which is at a higher risk for smaller-sized mammals of say less than 5kg.

Because of the high volume and continuous flow of gases through such prior art parallel Lack circuits, you cannot simply heat the moving gas in the inspired limb of the parallel Lack circuit, as is the case with a rebreathing circuit as disclosed in international patent publication no. WO2013/037004. This is because with the high volume and continuous flow of gas, the large volume of space in the inspired limb, and the large dead space of the Y-piece connector and endotracheal tube, it simply is not possible to effectively heat the gas as it rapidly passes through the parallel Lack circuit.

As a result, a major disadvantage of these prior art parallel Lack circuits is the potential for hypothermia in smaller-sized mammals, namely those having a mass of less than 5kg.

The present invention is to provide a connector piece for connecting the breathing tubes of an anaesthetic breathing circuit to an endotracheal tube for use with a mammal that overcomes at least one of the problems associated with the prior art.

The following publications disclose further examples of connectors known in the prior art.

"The role of 3D printing during COVID-19 pandemic: a review" by Longhitano Guilherme Arthue et al. published in Progress in Additive Manufacturing (XP093033581) discloses a modified "Charlotte" valve for connection to a snorkeling mask.

WO 2011/122964 A1 discloses a tracheal coupling for interfacing between a patient and a gases source.

US 2008/0011305 A1 discloses connectors for interconnecting a conduit of a fluid pressure device to an interface configured to deliver a fluid of the fluid pressure device to the patient.

WO 2018/236228 A1 discloses connectors for respiratory assistance systems.

WO 2016/022934 A1 discloses airway adapters and suction catheter systems.

### SUMMARY OF INVENTION

The invention is defined in the appended independent claim 1. Preferred embodiments are matter of the dependent claims.

A first aspect of the present invention consists of a connector piece for connecting the breathing tubes of an anaesthetic breathing circuit to an endotracheal tube for use with a mammal of no more than 15 kg mass, the connector piece having a gallery from which radiate first, second and third tube connectors, said first tube connector disposed between said second and third tube connectors, said first tube connector having a first longitudinal axis and being adapted to connect to a first of said breathing tubes, said second tube connector having a second longitudinal axis and being adapted to connect to a second of said breathing tubes, and said third tube connector having a third longitudinal axis and adapted to connect to said endotracheal tube, said third tube connector being disposed at an obtuse angle relative to said first tube connector with said third longitudinal axis intersecting said first longitudinal axis within said gallery, and said second tube connector disposed at an acute angle relative to said first tube connector with said second longitudinal axis intersecting said first longitudinal axis outside of said connector piece.

Preferably said third tube connector being disposed at an angle of about one hundred and ten degrees relative to said first tube connector and said second tube connector disposed at an angle of about thirty-five degrees relative to said first tube connector.

Preferably said third tube connector being disposed at an angle of about one hundred and forty-five degrees relative to said second tube connector.

Preferably said breathing tubes to which said first and second tube connectors attach each have an outside diameter of about 15mm.

Preferably said endotracheal tube which connects to said third tube connector has an internal diameter of about 5mm.

Preferably said gallery and said first and third tube connectors in combination provide an elbow-like change in direction within said connector piece that allows for orientation of breathing tubes relative to said endotracheal tube without the need of an elbow external of said connector piece, thus minimising mechanical dead space between said endotracheal tube and said breathing tubes.

Preferably an End-Tidal CO₂ adaptor is attached to either said first tube connector or said second tube connector and said End-Tidal CO₂ adaptor is for attaching a CO₂ sensor thereto.

Preferably said connector piece is for connecting an anaesthetic non-rebreathing circuit to an endotracheal tube for use with a smaller-sized mammal.

Preferably said anaesthetic non-rebreathing circuit is a Lack circuit having an adjustable pressure limited valve disposed at the waste gas port located at the expiratory end of said circuit, with fresh gas delivered by a first conduit from a vaporiser to said connector piece, said circuit having a heating arrangement for heating gas within said first conduit.

Preferably said Lack circuit is a parallel Lack circuit and said first conduit is one of two parallel breathing tubes.

Preferably said smaller-sized mammal has a mass of between 0.5 and 5kg.

A second aspect not according to the present invention consists of a connector piece for connecting the breathing tubes of an anaesthetic breathing circuit to an endotracheal tube for use with a small mammal, said connector piece having a gallery from which radiate first, second and third tube connectors, said first tube connector having a first longitudinal axis and being adapted to connect to a first of said breathing tubes, said second tube connector having a second longitudinal axis and being adapted to connect to a second of said breathing tubes, and said third tube connector having a third longitudinal axis and adapted to connect to said endotracheal tube, said gallery and said first and third tube connectors in combination provide an elbow-like change in direction within said connector piece, with said third longitudinal axis intersecting said first longitudinal axis within said gallery, and said second longitudinal axis intersecting said first longitudinal axis outside of said connector.

Preferably said elbow-like change in direction allows for orientation of breathing tubes relative to said endotracheal tube without the need of an elbow external of said connector piece, thus minimising mechanical dead space between said endotracheal tube and said breathing tubes.

Preferably said third tube connector being disposed at an obtuse angle relative to said first tube connector with said third longitudinal axis intersecting said first longitudinal axis within said gallery and said second tube connector disposed at an acute angle relative to said first tube connector with said second longitudinal axis intersecting said first longitudinal axis outside of said connector.

Preferably said third tube connector being disposed at an angle of about one hundred and ten degrees relative to said first tube connector and said second tube connector disposed at an angle of about thirty-five degrees relative to said first tube connector.

Preferably said third tube connector being disposed at an angle of about one hundred and forty-five degrees relative to said second tube connector.

Preferably said breathing tubes to which said first and second connectors attach each have an outside diameter of about 15mm.

Preferably said endotracheal tube which connects to said third tube connector has an internal diameter of about 5mm.

Preferably an End-Tidal CO₂ adaptor is attached to either said first tube connector or said second tube connector and said End-Tidal CO₂ adaptor is for attaching a CO₂ sensor thereto.

A third aspect of the present invention consists of a system for connecting an anaesthetic non-rebreathing circuit to an endotracheal tube for use with a mammal of no more than 15 kg mass, the system comprising: said connector piece of the present invention, the anaesthetic non-rebreathing circuit connected to the first tube connector and second tube connector, and the endotracheal tube connected to the third tube connector.

Preferably said anaesthetic non-rebreathing circuit is a Lack circuit having an adjustable pressure limited valve disposed at the waste gas port located at the expiratory end of said circuit, with fresh gas delivered by a first conduit from a vaporiser to said connector piece, said circuit having a heating arrangement for heating gas within said first conduit.

Preferably said Lack circuit is a parallel Lack circuit and said first conduit is one of two parallel breathing tubes.

### BRIEF DESCRIPTION OF DRAWINGS

Fig. 1(a) is a schematic upper perspective view of a cat positioned on its side, for a veterinary procedure, using a prior art conventional Y-piece connector to connect the endotracheal tube that is placed within the cat's mouth to the breathing tubes of an anaesthetic breathing circuit.
Fig. 1(b) is a schematic upper perspective view of a human infant positioned on its back, for abdominal surgery, using a prior art conventional Y-piece connector to connect the endotracheal tube that is placed within the cat's mouth to the breathing tubes of an anaesthetic breathing circuit.
Fig. 2(a) is a schematic upper perspective view of the cat shown in Fig.1 (a) where a right angle (90 degree) elbow is disposed between the endotracheal tube and the conventional Y-piece connector.
Fig. 2 (b) is a schematic upper perspective view of the human infant shown in Fig .1 (b) where a right angle (90 degree) elbow is disposed between the endotracheal tube and the conventional Y-piece connector.
Figs. 3(a), 3(b) and 3(c) schematic views depicting "dead space" within prior art endotracheal tube, paediatric Y-piece and 90° elbow, respectively.
Fig. 4 is a side view of a first embodiment of a connector piece for connecting the breathing tubes of an anaesthetic breathing circuit to an endotracheal tube for use with a mammal in accordance with the present invention.
Fig. 5 is a plan view of the connector piece shown in Fig. 4.
Fig. 6 is an end view of the connector piece shown in Fig. 4.
Fig. 7 is a perspective view of the connector piece shown in Fig. 4.
Fig. 8 is the side view of Fig. 4, with the orientation angles depicted.
Fig. 9 is a schematic view of the connector of Fig. 4 depicting a volume of certain "dead space" there within.
Fig. 10(a) is a schematic view of a cat positioned on its side, for a veterinary procedure, using the connector piece of Fig. 4 to connect the endotracheal tube that is placed within the cat's mouth to the breathing tubes of an anaesthetic breathing circuit.
Fig. 10(b) is a schematic view of a human infant positioned on its back, for abdominal surgery, using the connector piece of Fig. 4 to connect the endotracheal tube that is placed within the cat's mouth to the breathing tubes of an anaesthetic breathing circuit.
Fig. 11(a) is a schematic view of a prior art conventional Y-piece connector with an End-Tidal CO₂ connector and CO₂ sensor fitted to the expiratory limb.
Fig. 11(b) is a schematic view of the connector of Fig. 4 with an End-Tidal CO₂ connector and CO₂ sensor fitted to the expiratory limb.
Fig. 12 is a schematic of a prior art parallel Lack circuit, utilising a conventional "pop-off" exhalation valve and a conventional Y-piece.
Fig. 13 is a schematic of a first embodiment of parallel Lack circuit for a smaller-sized mammal utilising the connector piece of Fig. 4.
Figs 14a, 14b and 14c show the "End-inspiration" phase, "Early expiration" phase and End-expiration" phase of the parallel lack circuit shown in Fig. 13.

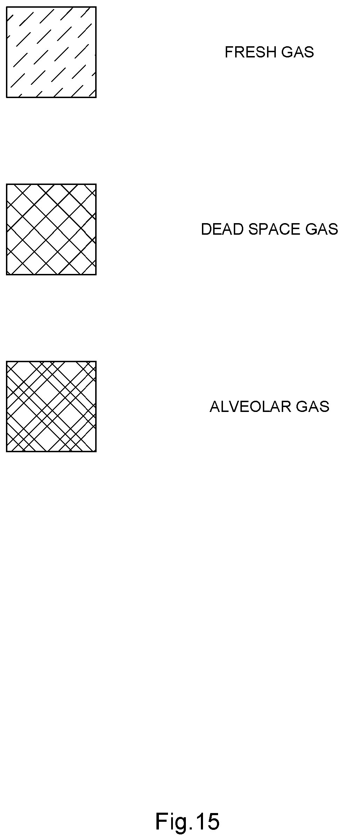

Fig 15. is the key of shaded areas "Fresh Gas", "Dead Space Gas" and "Alveolar Gas" shown in Figs 14a, 14b and 14c.

### BEST MODE OF CARRYING OUT THE INVENTION

In the present specification references to "small mammal", is a mammal of no more than 15kg mass. A "small mammal" may include but is not limited to any one of cats, dogs and human infants. Also, in the present specification references to a "smaller-sized mammal", is a mammal of no more than 5kg mass. A "smaller-sized mammal" may include but is not limited to cats and dogs.

In the present specification the term "mechanical dead space" (MDS) is used with reference to a mammal patient, such as a small mammal being anaesthetized. It is a volume within an endotracheal tube that is placed within the mouth of a small mammal and any connector piece which connects it to breathing tubes of an anaesthetic breathing circuit. This dead space is the volume within which the expired gas from the small mammal that contains CO₂, must be rebreathed before "fresh gas" not containing CO₂ is breathed in.

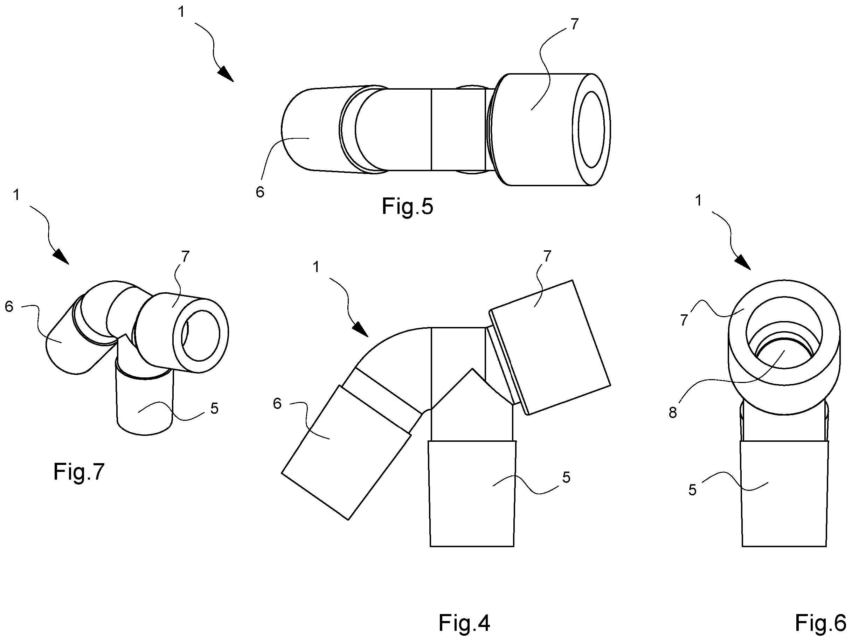

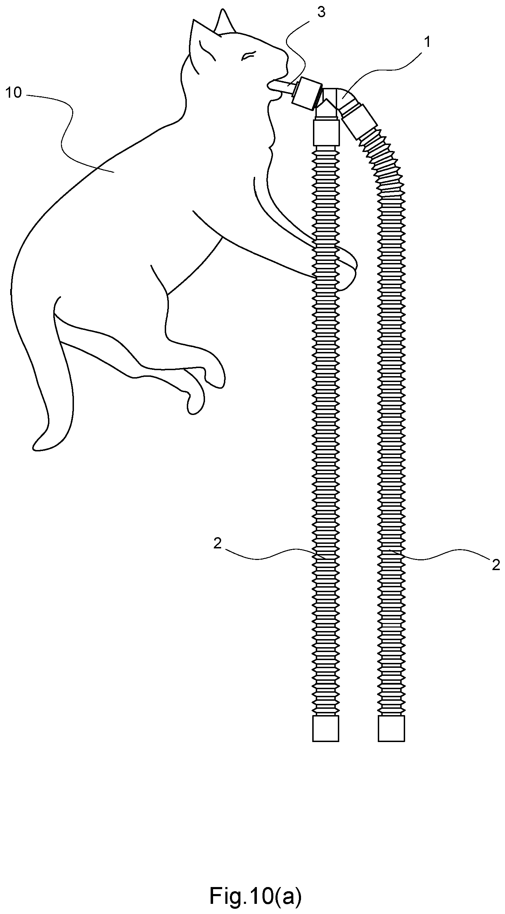

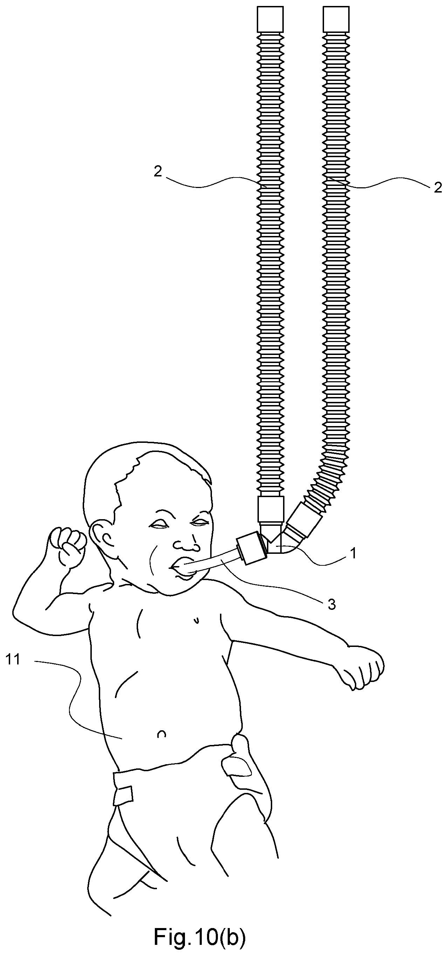

Figs 4 to 10(b) depict a connector piece 1 in accordance with a first embodiment of the present invention. Connector piece 1 is intended for procedures and surgery which require a small mammal, such as a cat 10 shown in Fig. 10(a), or human infant shown in Fig. 10(b) to be anaesthetized.

Connector piece 1, which is internally hollow, is for connecting breathing tubes 2 of anaesthetic equipment (not shown) that deliver anaesthetic in a breathable form (anaesthetic gas), to an endotracheal tube 3 that is placed within cat 10 via its mouth as shown Fig. 10(b).

In this embodiment connector piece 1 is preferably made of a plastic material, such as polycarbonate or polypropylene.

In this embodiment, connector piece 1 has three tube connectors (limbs), namely first connector 5, second connector 6 and third connector 7, all of which are tubular in form, and preferably have an outside diameter of 15mm.

Endotracheal tube 3 is made of vinyl and has an internal diameter of 5mm.

Disposed internally of connector piece 1, is a gallery 8 about which tube connectors 5, 6 and 7 radiate and interconnect. Connectors 5 and 6 are for connection to connector tubes (hoses) 2, whilst connector 7 is connected to endotracheal tube 3.

Connector 5 has a first longitudinal axis L₁ and is adapted to receive and connect to a first of the two breathing tubes 2. Connector 6 has a second longitudinal axis L₂ and is adapted to receive and connect to the other (second) of the two breathing tubes 2. Connector 7 having a third longitudinal axis L₃ is adapted to receive and connect to endotracheal tube 3.

In this embodiment, longitudinal axis L₃ of third connector 7 is disposed at an obtuse angle, namely one hundred-and-ten degrees, relative to first connector 5, with third longitudinal axis L₃ intersecting first longitudinal axis L₁ of first connector 5 within gallery 8. Second connector 6 is disposed at an acute angle, namely thirty-five degrees, relative to said first connector 5, with second longitudinal axis L₂ intersecting first longitudinal axis L₁ outside of connector piece 1 at point P. The angle between second connector 6 and third connector 7, namely the sum of one hundred-and-ten degrees and thirty-five degrees, is one hundred-and-forty-five degrees.

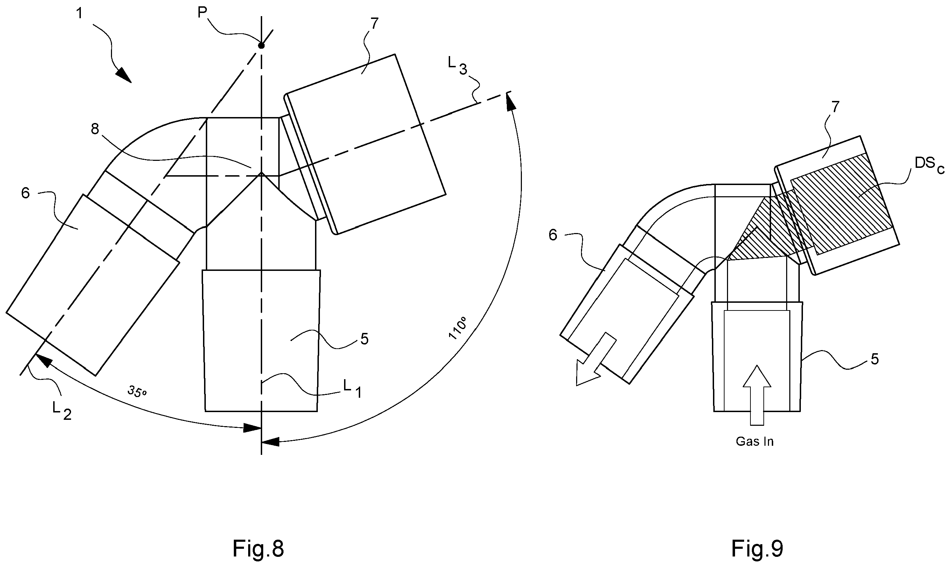

Connector piece 1 has an area of MDS shown as the shaded area DSc in Fig. 9.

Gallery 8 and first and third tube connectors 5,7 in combination, and due to the orientation of longitudinal axes L₁ and L₃, provide an "elbow-like" change in direction within connector piece 1.

As shown in Fig. 10(a), connector piece 1 of the present invention, with this abovementioned "elbow-like" change in direction within connector 1, allows for similar orientation of breathing tubes 2 relative to the cat 10, as the prior art arrangement, but has eliminated the need for the extra length of endotracheal tube, as required in the prior art shown in Fig. 1(a). Furthermore, the arrangement in Fig 10(a) has by the orientation to each other of connectors 5, 6 and 7 of connector piece 1, allowed for better directional placement of breathing tubes 2 without the need of an additional elbow 104, as was required in the prior art depicted Fig. 2(a). As, such connector piece 1 with its "elbow-like" change in direction eliminates both the kinking problem of the prior art depicted in Fig. 1(a) and minimises the MDS of both the prior art arrangements of Figs 1(a) and Figs. 2(a).

As shown in Fig. 10(b), connector piece 1 of the present invention allows for similar orientation of breathing tubes 2 relative to the human infant 11. In this embodiment, like that of cat 10 in Fig 10(a), connector piece 1 with its "elbow-like" change in direction eliminates both the kinking problem of the prior art depicted in Fig. 1(b) and minimises the MDS of both the prior art arrangements of Figs 1(b) and Figs. 2(b).

To highlight the minimization of MDS we make the following comparison of the present embodiment to that of the prior art in the following MDS calculations:

### Prior art Y Piece Connector arrangement Figs 1(a) & 1(b)

### Endotracheal Tube (ET) - 5mm ID

(typical for large 4kg cat Fig. 1(a) and Human Infant Fig 1(b))

| | |
|---|---|
| 3 cm projection from mouth ("standard" projection) | 0.6 ml |
| 4 cm projection from mouth to provide "bend" in tube (DS₃ in Fig. 3(a)) | 0.8 ml |
| MDS total for 7cm projection from mouth | **1.4 ml** |

### Standard Paediatric Y-Piece - 15mm OD

Total Volume = 14ml (mechanical dead space is a portion thereof - see Fig 3(b))
The breathing hose channels: π x 0.6252 x 2.8 = 3.44 ml
(each side) x 2 = **6.88ml**
Standard Paediatric Y-piece MDS = 14-6.88=7.12ml
   Standard Paediatric Y-Piece + 7cm Endotracheal tube projection has a
**MDS** = 7.12ml + 9.8ml = **8.52ml**

### Prior art Y Piece Connector with Elbow arrangement Figs 2(a) & 2(b)

90° elbow adaptor (AAS part No.8351) dead space shown as DS₄ in Fig. 3(c) = 9.8ml
Standard Paediatric Y-Piece + 90° Elbow + 3cm ET Tube projection
has an **MDS** = 7.12ml + 9.8ml + 0.6ml = **17.52ml**

### Present embodiment Connector Piece 1 arrangement Figs 8(a) and 8(b)

Connector piece 1 (grey shaded area dead space DSc) = 3.6ml
+ the 3cm ET Tube projection has an **MDS** = 3.6ml + 0.6ml = **4.2 ml**

This means that the MDS of the present embodiment is minimized to about 50% when compared to the Y-connector arrangement of Figs 1(a) and Figs 1(b), and to about 24% when compared to the elbow arrangement.

To better understand the improvement of the abovementioned preferred embodiment of present invention as described with reference to Figs. 4 to 10(b), it is important to consider the alveolar ventilation of the mammal.

Alveolar ventilation is the exchange of gas between the alveoli and the external environment. It is the process by which oxygen is brought into the lungs from the atmosphere and by which the carbon dioxide carried into the lungs in the mixed venous blood is expelled from the body.

### For Cat of mass 3kg

Normal Breath 10-15ml/kg =30-45 ml
Anatomic dead space (trachea etc) is about a third (say 33%) = about 10-15ml
So **net alveolar ventilation** = 20-30ml

### For Human infant of mass 5kg

Normal Breath 10-15ml/kg =50-75 ml
Anatomic dead space (trachea etc) =33% = about 15-25ml
So net alveolar ventilation = 35-50ml

Any anaesthetic connection dead space reduces alveolar ventilation. If severe, the mammal's blood CO₂ rises, anaesthetic level gets lighter (rebreathing dilutes inspired gas concentration), and the mammal may become hypoxic.

Anaesthetic MDS becomes less important once animals/infants (mammals) get over 10 to 15 kg, as they do not require a paediatric Y-piece of the earlier mentioned prior art. For anaesthetic MDS in a connector less than 5% is irrelevant and 10% is okay. However, MDS greater than that is problematic.

We now provide a **summary of MDS calculations** used for various sizes of small mammal.
**Prior art Paediatric Y piece (****Figs 1(a)** **&** **1(b)****)**
   = 7.12ml + 4cm extended ET tube: 0.8 ml = **7.92ml**
**Prior art Paediatric Y piece + elbow (****Figs 2(a)** **&** **2(b)****)**
   = 7.12ml + Elbow 9.8 ml = **16.92ml**
**Present embodiment Connector Piece 1 Figs 8(a) and 8(b) = 3.6ml**

Animal Pulmonary Ventilation: Tidal Volume (Vt)= 10ml/kg - 1/3 Anatomic Dead Space (DSa = nose, airway) + Machine DS (DSm) = net Alveolar Ventilation (V_{A})

So calculating this **V_{A} for various mammals of 3kg, 5kg, 10kg and 15 kg** mass is as follows:
**3kg x 10ml** = 30ml Vt - 10ml DSa = **20ml V_{A}**
**5kg x 10ml** = 50ml Vt - 16.7ml DSa = **33.3ml V_{A}**
**10kg x 10ml** = 100ml Vt - 33ml DSa = **67ml V_{A}**
**15kg x 10ml** = 150ml Vt - 50ml DSa = **100ml** V_{A}

**Table- Decrease of net Alveolar Ventilation (VA) for size of mammal**

| **Connector** | **3kg mammal (20ml V_{A})** | **5kg mammal (33.3ml V_{A})** | **10kg mammal (67ml V_{A})** | **15kg mammal (100ml V_{A})** |
|---|---|---|---|---|
| **Prior art Paediatric Y + extend ET tube 10ml MDS** | 20-7.92 = 12.08ml V_{A} =40% decrease | 33-7.92 = 25.08ml V_{A} =24% decrease | 67-7.92= 59.08ml V_{A} =12.6% decrease | 100-7.92 90ml V_{A} =8% decrease |
| **Prior art Paediatric Y + 90° elbow 10ml MDS** | 20-16.92 = 3.1ml V_{A} =85% decrease | 33-16.92 = 16.1ml V_{A} =51 % decrease | 67-16.92= 50.1 ml V_{A} =25% decrease | 100-16.92= 83.1 ml V_{A} = 17% decrease |
| **embodiment Connector Piece 1 3.6ml MDS** | 20-3.6 = 16.4ml V_{A} =18% decrease | 33-3.6 = 29.4ml V_{A} = 11% decrease | 67-3.6= 63.4ml V_{A} = 5.4% decrease | 100-3.6 96.4 ml V_{A} =3.6% decrease |

So as can be seen from this abovementioned table, connector piece 1 significantly minimizes the decrease of Alveolar Ventilation (V_{A}) when compared to the prior art, and this minimization is more significant in the smaller mass (sized) mammals.

In the above referenced embodiment, the small mammals referred to are cats and human infants, however the invention of the present embodiment could be on used on any small mammals including but not limited to dogs, rabbits, macropods, monkeys, and chimpanzees.

It should be understood, that the smaller the mammal, then the size of mechanical dead space has a greater impact, so the real advantages of minimizing MDS by use of connector piece 1, is even greater the smaller the mass of the mammal. This means it is particularly advantageous for use with anaesthetic breathing circuits for domesticated cats whose typical adult mass is 3.5-4.5kg, and smaller breed dogs, such as Chihuahuas and Pomeranians for example.

Referring to Fig. 4, what should be understood is that whilst connector piece 1 is shown with third connector 7 disposed at an obtuse angle, namely one hundred-and-ten degrees, relative to first connector 5, and second connector 6 is disposed at an acute angle, namely thirty-five degrees, relative to said first connector 5, these angles could vary. When they vary, it is preferable that the angle between second connector 6 and third connector 7 remain at about one hundred-and-forty-five degrees. So, in another not shown embodiment if the obtuse angle between third connector 7 and first connector 5 is increased by five degrees to that shown, namely to one hundred-and-fifteen degrees, then the acute angle between second connector 6 and first connector 5 should preferably be decreased by about five degrees to thirty degrees, thereby maintaining the sum of those two angles between second connector 6 and third connector 7 at about one hundred-and-forty-five degrees. By doing so the orientation of the connectors 5,6,7 maintain a preferable "elbow-like" change in direction within connector piece 1. Likewise, in a further not shown embodiment, if the obtuse angle between third connector 7 and first connector 5 is decreased by five degrees to that shown, namely to one hundred-and-five degrees, then the acute angle between second connector 6 and first connector 5 should preferably be increased by about five degrees to forty degrees, thereby maintaining the sum of those two angles between second connector 6 and third connector 7 at about one hundred-and-forty-five degrees. Likewise, by doing so the orientation of the connectors 5,6,7 maintain a preferable "elbow-like" change in direction within connector piece 1.

The abovementioned embodiment of present invention is also advantageous when used with End-Tidal CO₂ (ETCO₂) adaptors for smaller mammals, particularly the mid-sized smaller animals from 5-10kg.

To best show this advantage, it is best to understand the prior art. Any anaesthetic connection dead space reduces alveolar ventilation. In smaller animals/infants this problem is important because rebreathing CO₂ causes the animals blood CO₂ to rise which increases circulating catecholamines, heart rate, blood pressure and cardiac output. Rebreathing also dilutes the inspired concentration of anaesthetic so anaesthetic level gets lighter and can lead to hypoxia.

The commonest way to continuously measure the CO₂ level is by end-expired CO₂ sampling (ETCO₂ = equivalent to alveolar gas sampling) using infrared (IR) gas analysis (CO₂ gas absorbs infra-red light in a concentration - dependent manner). Whilst there are two ways of doing this, namely, side-stream sampling or main-stream sampling, the latter is more accurate with smaller mammals.

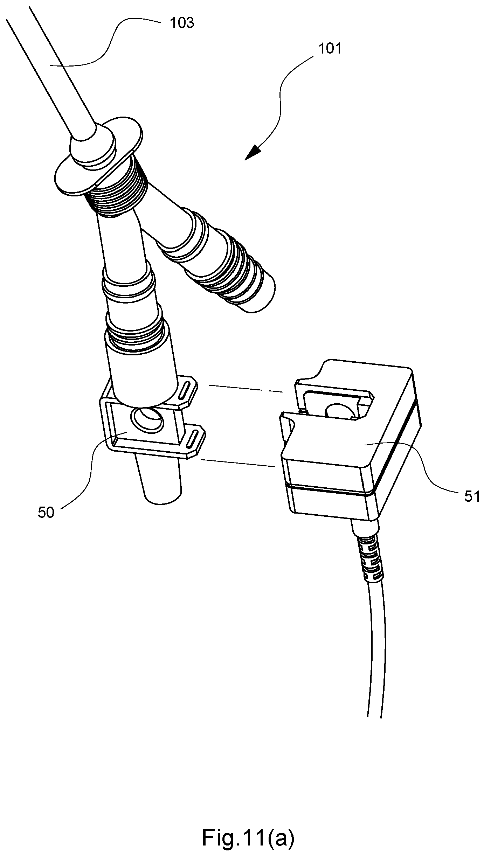

Prior art main-stream sampling for smaller mammals of 5-10kg mass as shown Fig 11(a), requires adding a sample ETCO₂ connector 50 between endotracheal tube 103 and Y-Piece connectors 101 (earlier shown in Figs 1(a) and 1(b)), that the animal/human breaths through. Connector 50 is attached to the expiratory limb of Y-piece connector 101. Connector 50 has a side window. A CO₂ sensor 51 incorporating an IR spectrophotometer is attached to connector 50 and measures the CO₂ level in real time across the window as the animal breathes in and out. This system is more accurate in small animals and humans where the breath size is small and is preferable over that of the main-stream sampling system, however it does have the potential of adding MDS.

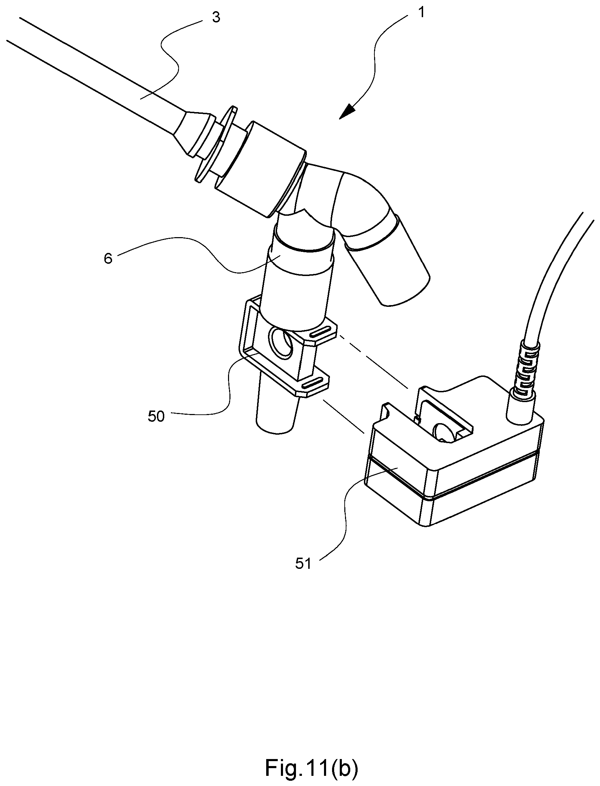

Connector 50 and sensor 51, can be used with connector piece 1, connected to endotracheal tube 3 of the earlier describe embodiment, as shown in Fig 11(b). Connector 50 is attached to the expiratory limb (second connector 6). In this arrangement, because the MDS has been minimized by use of connector piece 1, the placement of connector 50 and sensor 51 at the expiratory limb (second connector 6), allows for a more accurate CO₂ sample for the smallest patient for which this set up is used, namely for small mammals of mass 5kg to 10kg.

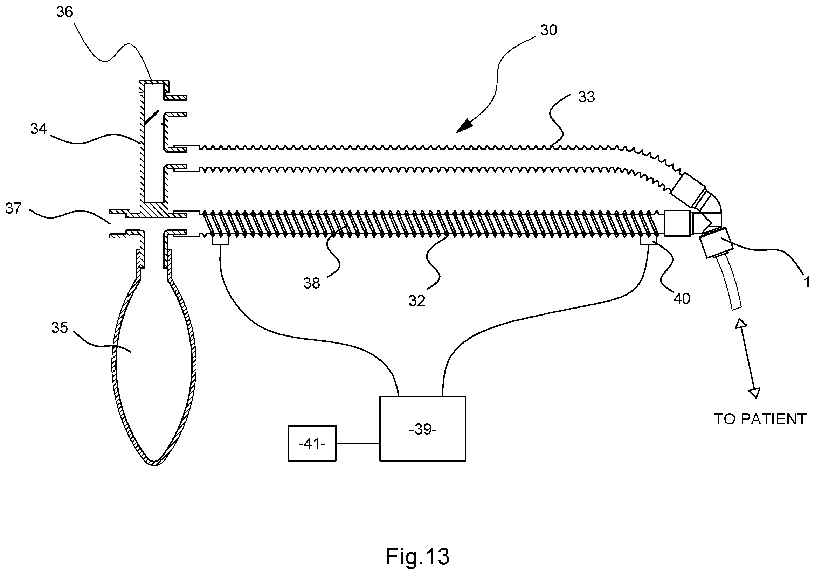

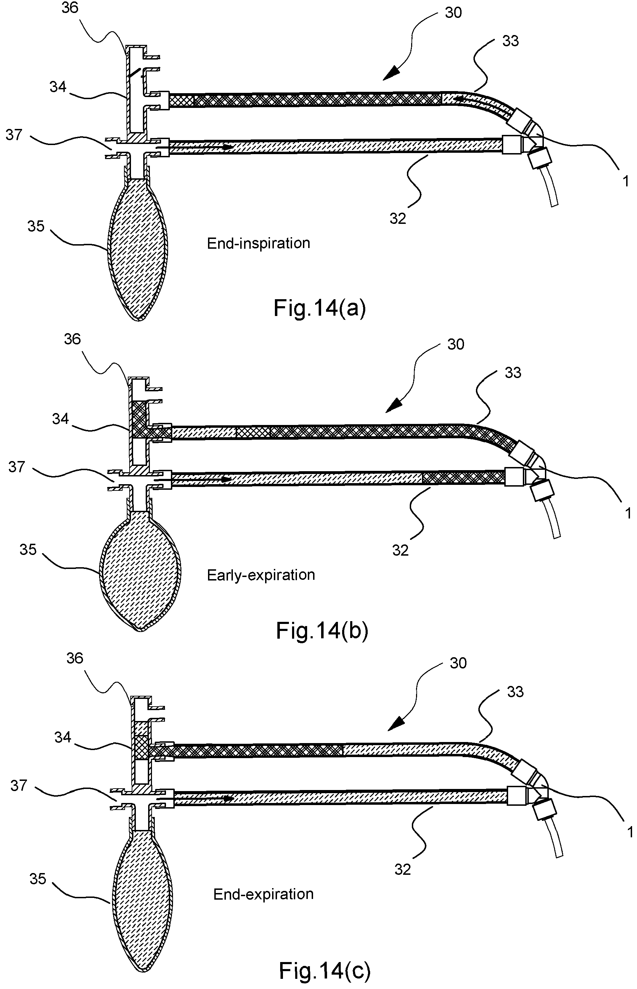

Figs. 13 and 14a, 14b and 14c depict a parallel Lack circuit 30. Connector piece 1 is shown at the patient end, intended to connect to an endotracheal tube (not shown) of a mammal. Extending from connector 1 are two substantially parallel tubes, identified as fresh gas inspiratory tube (conduit) 32 and reservoir tube (conduit) 33 which extend to circuit block 34 at the "vaporiser end" of circuit 30. Circuit block 34 is fitted with a reservoir bag 35 and exhalation valve 36 at the waste gas port and has an inlet 37 through which fresh gas (containing anaesthetic agent) is delivered from a vaporiser (not shown). Circuit block 34 is blanked off between the inlet 37 side and exhalation valve 36 so that there is no connection therebetween within block 34.

Exhalation valve 36 is a "fail-safe" adjustable pressure-limiting (APL) valve that allows for efficient low gas flows. This APL valve 36 is capable having the flow adjusted in gradations. An APL valve 36 that is suitable has 2cmH₂O positive end-expiratory pressure (PEEP) when fully open and graduated to 25cmH₂O fully closed. From 2cmH₂O to 25cmH₂O is a graduated scale with linear increase in PEEP as it progresses from full open to full closed.

One such APL Valve 36 which is suitable is the "Paediatric APL Valve" by Intersurgical^{™}.

When this Paediatric APL Valve by Intersurgical^{™} is fully open:
- resistance to flow = 0.4cm H₂O at 3L/min continuous flow; and
it is actuated by expiratory force/pressure of less than 1cm H₂O (force required to overcome static opening resistance)

Valve fully closed:
- resistance to flow = 27cmH₂O at 4L/min (30-31cm H₂O at 10L/min) - 4L/min/200ml/kg/min = 20Kg mammal

This Paediatric APL Valve by Intersurgical^{™} has linear performance from open to closed, so increasing PEEP from 0.4cm H₂O to 27cm H₂O

Fresh gas inspiratory tube 32 preferably has a twelve millimetre (12 mm) inside diameter, and preferably is no longer than 1.6m. Inspiratory tube 32 is provided with a "heating arrangement", comprising a heating element 38 and a thermostatic control unit 39 powered by a DC supply 41. Thermostatic control unit 39 controls the heat delivered to heating element 38. In Fig. 13 heating element 38 is a spiral wound element extending along a substantial portion of inspiratory tube 32. However, in an alternative arrangement heating element 38 could be a strip element extending along a substantial portion of inspiratory tube 32.

You could use sixteen millimeter (16mm) inside diameter tube for the fresh gas inspiratory tube 32. However, it is possible to warm the inspired gas more effectively with 12mm ID tube 32, compared to 16mm at the same O₂ flows. The 12 mm ID tube is preferred for the small-sized mammals as the fresh gas is "closer" to heater element 3,8 so better and more rapid warming is achieved. A 12mm tube which is 1.6m long holds 180ml gas, which is enough for a 12-18kg mammal.

One suitable inspiratory tube 32 in combination with the "heating arrangement" is the twelve millimetre (12 mm) inside diameter heated tube (which includes heating element 38) of the DARVALL^{™} Warm Air Starter Kit, the control unit 39 being the DARVALL^{™} WARM AIR INSPIRED control unit.

This thermostatic control unit 39, also includes a temperature sensor 40 to sense the temperature of the heated fresh gas passing through inspiratory tube 32, and a temperature display (not shown).

The operation of parallel Lack circuit 30, and its suitability for smaller-sized mammals will now be described with reference to Figs 14a, 14b and 14c, which depict the location of Fresh Gas, Dead Space Gas and Alveolar Gas during the respective "End-inspiration", "Early expiration" and End-expiration" phases.

Dead Space Gas is primarily the gas made up of oxygen (O₂) and carbon dioxide (CO₂) gasses that are not exchanged across the alveolar membrane in the respiratory tract. This Dead Space Gas is from both MDS and the patient's anatomic dead space.

Alveolar Gas is the gas expired by the patient which contains CO₂.
- APL valve 36 provides a gradually increasing resistance as it is opened or closed to apply some PEEP, typically 2 to 4 cm H₂0, to replace the simple open-close "pop-off" waste gas outlet valve of the prior art. Where the fresh gas flow through inspiratory tube 32 balances the PEEP on APL valve 36, reservoir bag 35 stays "full" at lower fresh gas flows, see Figs 14b and 14c, preventing rebreathing of CO₂ but also reducing the fresh gas flow to a minimum flow of about 200ml/min (sufficient for a 1kg to 2kg mammal).
- The use of a 1.6m length of 12mm ID heated smooth wall tube for inspiration tube 32 with a volume of about 180ml, means that effectively more than one breath is "stored" in the inspiratory limb, for animals up to 12kg-18kg (10-15ml/kg/breath) permitting effective warming to 45°C at the connector piece (patient) hose end, at fresh gas flows of 200ml/min to 2000ml/min. The temperature of the fresh gas will effectively drop to about 35°C by the time it is inspired by the patient.
- By using connector piece 1, which has an MDS of 3.6 ml, this parallel Lack circuit 30 can be used on mammals with a breath size as small as 7ml, and this connector piece 1, only increases the dead space from 30% (normal) to 50% (acceptable) so permitting safe, low flow use of parallel Lack circuit 30 on mammals from 0.5 to 2kg and enable effective warming of the inspired gas to 45°C at the patient connection.

Trials using this parallel Lack circuit 30 incorporating connector piece 1 have shown that for a smaller-sized mammal of 1kg and with a "Fresh Gas" flow of 200ml/min from a Darvall Vaporiser (as disclosed in International Patent Publication No. WO2020/146919) that no rebreathing of CO₂ (Alveolar Gas) occurred. Furthermore, this circuit 30 was tested as low as 100ml/min without rebreathing of CO₂. This occurs because of the low inlet gas flow is balanced to the outlet gas flow by APL valve 36 in combination with the minimised dead space provided by connector piece 1. Furthermore because of the lower flow rate, the volume of fresh gas is "almost stationary" in the inspired limb of circuit 30, with each breath is as high as 180mls, this fresh gas can be heated sufficiently as it passes through tube 32, so that the smaller-sized mammal of 1kg mass receives warm air heated to 45°C thereby minimising the risk of hypothermia.

The terms "comprising" and "including" (and their grammatical variations) as used herein are used in an inclusive sense and not in the exclusive sense of "consisting only of".

## Claims

1. A connector piece (1) for connecting the breathing tubes (2) of an anaesthetic breathing circuit to an endotracheal tube (3) for use with a mammal of no more than 15 kg mass, the connector piece (1) having a gallery (8) from which radiate first (5), second (6) and third tube connectors (7),
said first tube connector (5) disposed between said second (6) and third tube connectors (7),
said first tube connector (5) having a first longitudinal axis and being adapted to connect to a first of said breathing tubes (2),
said second tube connector (6) having a second longitudinal axis and being adapted to connect to a second of said breathing tubes (2), and
said third tube connector (7) having a third longitudinal axis and adapted to connect to said endotracheal tube (3),
said third tube connector (7) being disposed at an obtuse angle relative to said first tube connector (5) with said third longitudinal axis intersecting said first longitudinal axis within said gallery (8), and
said second tube connector (6) disposed at an acute angle relative to said first tube connector (5) with said second longitudinal axis intersecting said first longitudinal axis outside of said connector piece (1).

2. The connector piece (1) as claimed in claim 1, wherein said third tube connector (7) being disposed at an angle of about one hundred and ten degrees relative to said first tube connector (5) and
said second tube connector (6) disposed at an angle of about thirty-five degrees relative to said first tube connector (5).

3. The connector piece (1) as claimed in claim 1, wherein said third tube connector (7) being disposed at an angle of about one hundred and forty-five degrees relative to said second tube connector (6).

4. The connector piece (1) as claimed in any of claims 1 to 3, wherein said breathing tubes (2) to which said first (5) and second tube connectors (6) attach each have an outside diameter of about 15mm.

5. The connector (1) piece as claimed in any of claims 1 to 4 wherein said endotracheal tube (3) which connects to said third tube connector (7) has an internal diameter of about 5mm.

6. The connector piece (1) as claimed in 1, wherein said gallery (8) and said first (5) and third tube connectors (7) in combination provide an elbow-like change in direction within said connector piece that allows for orientation of the breathing tubes (2) relative to said endotracheal tube (3) without the need of an elbow external of said connector piece, thus minimising mechanical dead space between said endotracheal tube (3) and said breathing tubes (2).

7. The connector piece (1) as claimed in any of claims 1 to 6, wherein an End-Tidal CO₂ adaptor is attached to either said first tube connector (5) or said second tube connector (7) and
said End-Tidal CO₂ adaptor (50) is for attaching a CO2 sensor (51) thereto.

8. A system for connecting an anaesthetic non-rebreathing circuit to an endotracheal tube for use with a mammal of no more than 15 kg mass, the system comprising:
the connector piece (1) as claimed in claim 1,
the anaesthetic non-rebreathing circuit connected to the first tube connector (5) and second tube connector (7), and
the endotracheal tube (3) connected to the third tube connector (7).

9. The system as claimed in claim 8, wherein said anaesthetic non-rebreathing circuit is a Lack circuit (30) having an adjustable pressure limited valve (36) disposed at the waste gas port located at the expiratory end of said circuit, with fresh gas delivered by a first conduit from a vaporiser to said connector piece (1), said circuit having a heating arrangement for heating gas within said first conduit.

10. The system as claimed in claim 9, wherein said Lack circuit (30) is a parallel Lack circuit, and said first conduit is one of two parallel breathing tubes (2).

## Patentansprüche

1. Verbindungsstück (1) zum Verbinden der Beatmungsschläuche (2) eines Anästhesie-Beatmungskreislaufs mit einem Endotrachealtubus (3) zur Verwendung bei einem Säugetier mit einem Körpergewicht von höchstens 15 kg, wobei das Verbindungsstück (1) einen Kanal (8) aufweist, von dem ein erster (5), ein zweiter (6) und ein dritter (7) Schlauchverbinder abzweigen.
wobei der erste Schlauchverbinder (5) zwischen dem zweiten (6) und dem dritten Schlauchverbinder (7) angeordnet ist,
der erste Schlauchverbinder (5) eine erste Längsachse hat und zum Verbinden mit einem ersten der Beatmungsschläuche (2) eingerichtet ist,
der zweite Schlauchverbinder (6) eine zweite Längsachse hat und zum Verbinden mit einem zweiten der Beatmungsschläuche (2) eingerichtet ist, und
der dritte Schlauchverbinder (7) eine dritte Längsachse hat und zum Verbinden mit dem Endotrachealtubus (3) eingerichtet ist,
der dritte Schlauchverbinder (7) in einem stumpfen Winkel relativ zu dem ersten Schlauchverbinder (5) angeordnet ist, und dabei die dritte Längsachse die erste Längsachse innerhalb des Kanals (8) schneidet, und
der zweite Schlauchverbinder (6) in einem spitzen Winkel relativ zu dem ersten Schlauchverbinder (5) angeordnet ist, und dabei die zweite Längsachse die erste Längsachse außerhalb des Verbindungsstücks (1) schneidet.

2. Verbindungsstück (1) nach Anspruch 1, wobei der dritte Schlauchverbinder (7) in einem Winkel von ungefähr 110° relativ zu dem ersten Schlauchverbinder (5) angeordnet ist, und
der zweite Schlauchverbinder (6) in einem Winkel von ungefähr 35° relativ zu dem ersten Schlauchverbinder (5) angeordnet ist.

3. Verbindungsstück (1) nach Anspruch 1, wobei der dritte Schlauchverbinder (7) in einem Winkel von ungefähr 145° relativ zu dem zweiten Schlauchverbinder (6) angeordnet ist.

4. Verbindungsstück (1) nach einem der Ansprüche 1 bis 3, wobei die Beatmungsschläuche (2), an die der erste (5) und der zweite Schlauchverbinder (6) angeschlossen sind, jeweils einen Außendurchmesser von ungefähr 15 mm haben.

5. Verbindungsstück (1) nach einem der Ansprüche 1 bis 4, wobei der Endotrachealtubus (3), mit dem der dritte Schlauchverbinder (7) verbunden ist, einen Innendurchmesser von ungefähr 5 mm hat.

6. Verbindungsstück (1) nach Anspruch 1, wobei der Kanal (8) und der erste (5) sowie der dritte Schlauchverbinder (7) in Kombination eine knieförmige Richtungsänderung innerhalb des Verbindungsstücks bewirken, die Ausrichtung der Beatmungsschläuche (2) relativ zu dem Endotrachealtubus (3) ermöglicht, ohne dass ein Knie außerhalb des Verbindungsstücks erforderlich ist, wodurch mechanischer Totraum zwischen dem Endotrachealtubus (3) und den Beatmungsschläuchen (2) minimiert wird.

7. Verbindungsstück (1) nach einem der Ansprüche 1 bis 6, wobei ein EtCO₂-Messadapter entweder an dem ersten Schlauchverbinder (5) oder dem zweiten Schlauchverbinder (7) angeschlossen ist, und
der EtCO₂-Messadapter (50) zum Anschließen eines CO₂-Sensors (51) daran dient.

8. System zum Verbinden eines Anästhesie-Kreislaufs ohne Rückatmung mit einem Endotrachealtubus zur Verwendung bei einem Säugetier mit einem Körpergewicht von höchstens 15 kg, wobei das System umfasst:
das Verbindungsstück (1) nach Anspruch 1,
den Anästhesie-Kreislauf ohne Rückatmung, der mit dem ersten Schlauchverbinder (5) und dem zweiten Schlauchverbinder (7) verbunden ist, sowie
den Endotrachealtubus (3), der mit dem dritten Schlauchverbinder (7) verbunden ist,

9. System nach Anspruch 8, wobei der Anästhesie-Kreislauf ohne Rückatmung ein Lack-Kreislauf (30) mit einem einstellbaren Druckbegrenzungsventil (36) ist, das an dem Abgasanschluss angeordnet ist, der sich am Ausatmungsende des Kreislaufs befindet, wobei Frischgas über eine erste Leitung von einem Verdampfer dem Verbindungsstück (1) zugeführt wird, und wobei der Kreislauf eine Heizanordnung zum Erwärmen von Gas innerhalb der ersten Leitung aufweist.

10. System nach Anspruch 9, wobei der Lack-Kreislauf (30) ein paralleler Lack-Kreislauf ist und die erste Leitung einer von zwei parallelen Beatmungsschläuchen (2) ist.

## Revendications

1. Pièce raccord (1) pour relier les tubes respiratoires (2) d'un circuit respiratoire anesthésique à un tube endotrachéal (3), pour utilisation avec un mammifère d'une masse ne dépassant pas 15 kg, la pièce raccord (1) présentant une galerie (8) à partir de laquelle rayonnent des premier (5), deuxième (6) et troisième raccords de tube (7), ledit premier raccord de tube (5) étant disposé entre lesdits deuxième (6) et troisième raccords de tube (7),
ledit premier raccord de tube (5) présentant un premier axe longitudinal et étant adapté pour être raccordé à un premier desdits tubes respiratoires (2),
ledit deuxième raccord de tube (6) présentant un deuxième axe longitudinal et étant adapté pour être raccordé à un second desdits tubes respiratoires (2), et
ledit troisième raccord de tube (7) présentant un troisième axe longitudinal et étant adapté pour être raccordé audit tube endotrachéal (3),
ledit troisième raccord de tube (7) étant disposé à un angle obtus par rapport audit premier raccord de tube (5), ledit troisième axe longitudinal recoupant ledit premier axe longitudinal à l'intérieur de ladite galerie (8), et
ledit deuxième raccord de tube (6) étant disposé à un angle aigu par rapport audit premier raccord de tube (5), ledit deuxième axe longitudinal recoupant ledit premier axe longitudinal à l'extérieur de ladite pièce raccord (1).

2. Pièce raccord (1) selon la revendication 1, dans laquelle ledit troisième raccord de tube (7) est disposé à un angle d'environ cent dix degrés par rapport audit premier raccord de tube (5), et
ledit deuxième raccord de tube (6) étant disposé à un angle d'environ trente-cinq degrés par rapport audit premier raccord de tube (5).

3. Pièce raccord (1) selon la revendication 1, dans laquelle ledit troisième raccord de tube (7) est disposé à un angle d'environ cent quarante-cinq degrés par rapport audit deuxième raccord de tube (6).

4. Pièce raccord (1) selon l'une quelconque des revendications 1 à 3, dans laquelle lesdits tubes respiratoires (2) auxquels lesdits premier (5) et deuxième (6) raccords de tube se fixent présentent chacun un diamètre extérieur d'environ 15 mm.

5. Pièce raccord (1) selon l'une quelconque des revendications 1 à 4, dans laquelle ledit tube endotrachéal (3) qui est raccordé audit troisième raccord de tube (7) présente un diamètre intérieur d'environ 5 mm.

6. Pièce raccord (1) selon la revendication 1, dans laquelle ladite galerie (8) et lesdits premier (5) et troisième raccords de tube (7) fournissent en combinaison un changement de direction de type coude à l'intérieur de ladite pièce raccord qui permet une orientation des tubes respiratoires (2) par rapport audit tube endotrachéal (3) sans avoir besoin d'un coude externe à ladite pièce raccord, en minimisant ainsi l'espace mort mécanique entre ledit tube endotrachéal (3) et lesdits tubes respiratoires (2).

7. Pièce raccord (1) selon l'une quelconque des revendications 1 à 6, dans laquelle un adaptateur de CO2 End-Tidal est fixé soit audit premier raccord de tube (5) soit audit deuxième raccord de tube (7), et
ledit adaptateur de CO2 End-Tidal (50) est destiné à la fixation d'un capteur de CO2 (51) à celui-ci.

8. Système pour raccorder un circuit anesthésique non respiratoire à une sonde endotrachéale pour une utilisation avec un mammifère d'une masse ne dépassant pas 15 kg, le système comprenant :
la pièce raccord (1) selon la revendication 1,
le circuit anesthésique sans respiration raccordé au premier raccord de tube (5) et au deuxième raccord de tube (7), et
le tube endotrachéal (3) raccordé au troisième raccord de tube (7).

9. Système selon la revendication 8, dans lequel ledit circuit anesthésique sans respiration est un circuit Lack (30) présentant une valve de limitation de pression réglable (36) disposée au niveau de l'orifice de gaz résiduaires situé à l'extrémité d'expiration dudit circuit, avec du gaz frais délivré par un premier conduit à partir d'un vaporisateur vers ladite pièce raccord (1), ledit circuit présentant un agencement de chauffage pour chauffer du gaz à l'intérieur dudit premier conduit.

10. Système selon la revendication 9, dans lequel ledit circuit Lack (30) est un circuit Lack parallèle,et ledit premier conduit est l'un de deux tubes respiratoires parallèles (2).
